(19) Europäisches Patentamt
European Patent Office
Office européen des brevets

(11) Veröffentlichungsnummer: **0 440 078 B1**

(12) # EUROPÄISCHE PATENTSCHRIFT

(45) Veröffentlichungstag der Patentschrift: **19.04.95**

(51) Int. Cl.[6]: **C07H 15/10**, C07H 15/18, C07H 15/26

(21) Anmeldenummer: **91100793.8**

(22) Anmeldetag: **23.01.91**

(54) **Verfahren zur Herstellung von Retinylglykosiden und Zwischenprodukte für dieses Verfahren.**

(30) Priorität: **02.02.90 DE 4003094**

(43) Veröffentlichungstag der Anmeldung:
**07.08.91 Patentblatt 91/32**

(45) Bekanntmachung des Hinweises auf die Patenterteilung:
**19.04.95 Patentblatt 95/16**

(84) Benannte Vertragsstaaten:
**CH DE FR GB LI NL**

(56) Entgegenhaltungen:
**DE-A- 1 046 612**
**DE-A- 1 068 710**
**US-A- 4 855 463**

**LIEBIGS ANN. CHEM. 1989, Seiten 79 - 81 I.E. ACKERMANN ET AL. 'Preparation of new terpenyl glucopyranosides by a modified K nigs-Knorr procedure'**

**CHEMICAL ABSTRACTS, vol. 73, 1970, Columbus, Ohio, US; abstract no. 69834z, N. TAKABAYASHI ET AL. 'Vitamin A glucose ether'**

**BIOCHEM. J. Bd. 244, 1987, Seiten 231 - 234 A.B. BARUA ET AL. 'Chemical synthesis and growth-promoting activity of all-trans-retinyl beta-D-glucuronide'**

(73) Patentinhaber: **BASF Aktiengesellschaft**
**Carl-Bosch-Strasse 38**
**D-67063 Ludwigshafen (DE)**

(72) Erfinder: **Von Deessen, Ulrich, Dr.**
**Im Lammsbauch 33**
**W-6720 Speyer (DE)**
Erfinder: **Paust, Joachim, Dr.**
**Ringstrasse 3**
**W-6708 Neuhofen (DE)**
Erfinder: **Kaiser, Klaus, Dr.**
**Wittelsbacher Strasse 18**
**W-6730 Neustadt (DE)**
Erfinder: **Indest, Helmut, Dr.**
**Am Moenchgarten 34**
**W-6940 Weinheim (DE)**

EP 0 440 078 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Retinylglykosiden, insbesondere von Retinylglucuroniden sowie von Retinyl-β-D-glucopyranosid, sowie neue Zwischenprodukte für dieses Verfahren.

Stand der Technik

Kohlenhydrate, insbesondere die Glykoside, sind von besonderer Bedeutung für die Zell-Zell-Interaktion. Sie fungieren als Rezeptoren für Hormone, Proteine, Bakterien und Viren. Desweiteren determinieren sie die antigenen Eigenschaften von Zellen (vgl. beispielsweise H. Paulsen, Angew. Chem. 94 (1982) 184-201).

Insbesondere das Interesse an Glucuroniden ist in den letzten Jahren stark gewachsen, da erkannt wurde, daß diese Substanzen ungewöhnliche biologische Aktivitäten besitzen (siehe beispielsweise F.M. Kaspersen et al. in Xenobiotica 17 (1987), 1451-1471).

So fördern beispielsweise Retinylglucuronide das Größenwachstum bei Ratten, inhibieren entartete Tumorzellen und spielen eine Rolle bei der Zelldifferenzierung (vgl. J.A. Olson in Chemica Scripta 27 (1987) 179-183 und A.B. Barua et al. in Biochem. J. 252 (1988) 415-420).

Für die Herstellung von Retinylglucuroniden sind bisher im wesentlichen 2 Verfahren bekannt. Gemäß einer Arbeit von Barua et al. in Biochem. J. 244 (1987), 231-234, erhält man durch Umsetzen von All-trans-Retinol mit 2,3,4-Tri-O-acetyl-1-bromo-1-deoxy-β-D-glucopyran-uronsäuremethylester in Diethylether in Gegenwart von $Ag_2CO_3$ und anschließende Hydrolyse den Retinylglucuronsäuremethylester. Nachteilig an diesem Verfahren ist, daß die erzielbaren Ausbeuten bei nur etwa 15% der Theorie liegen. In einer früheren Beschreibung dieses Verfahrens (vgl. US 4,855,463) wurden von Barua et al zwar Ausbeuten von etwa 60 % angegeben, doch konnten diese Ergebnisse nicht reproduziert werden.

Gemäß dem verfahren von US 4 457 918 erhält man Glykoside der Vitamine A, E und K durch Umsetzen der entsprechenden Vitamine mit einem acylierten Kohlenhydrat oder einem acylierten glykosidischen Polymer, das in dem Kohlenhydrat oder dem endständigen glykosidischen Rest in 1-Stellung eine geeignete Abgangsgruppe enthält in einem inerten nichtpolaren Lösungsmittel in Gegenwart von Silbertrifluormethansulfonat und 2,4,6-Trimethylpyridin und anschließende Deacylierung der glykosidischen Reste mittels Basen. Auch die bei diesem Verfahren erzielten Ausbeuten sind unbefriedigend. Ein weiterer Nachteil der bekannten Verfahren ist, daß man aufgrund der großen Empfindlichkeit des Retinols gegenüber Säuren bei der Wahl der Glykosidierungskatalysatoren und das Schutzgruppenmusters im Kohlenhydratteil stark eingeschränkt ist.

Es war daher die Aufgabe der Erfindung, ein Verfahren zu entwickeln, mit dessen Hilfe man auf möglichst einfache Weise Glykoside des Retinols (Vitamin A) in guten Ausbeuten herstellen kann.

Es wurde nun überraschenderweise gefunden, daß man Retinylglykoside in sehr guten Ausbeuten erhalten kann, wenn man zunächst ein 4-Hydroxy-2-methyl-2-buten-1-al mit geschützter Aldehydgruppe glykosidisch an eine acylierte Zuckereinheit bindet und das durch anschließende Abspaltung der Aldehydschutzgruppe erhaltene in 4-Stellung glykosidisch mit der acylierten Zuckereinheit verknüpfte 4-Hydroxy-2-methyl-2-buten-1-al in einer Wittig-Reaktion mit einem β-Jonylidenethyltriphenylphosphoniumsalz umsetzt.

Es war zwar schon aus DE-A- 1 046 612 bzw. DE-A- 1 068 710 bekannt, daß man einfache Retinol-Derivate, wie Vitamin-A-ester und Vitamin-A-ether durch Wittig-Reaktion von veresterten bzw. veretherten γ-Hydroxy-α-methyl-crotonaldehyd-Derivaten mit β-Jonyliden-triphenylphosphonium-Derivaten herstellen kann, jedoch war nicht zu erwarten, daß sich die Glykosylierung von 4-Hydroxy-2-methyl-2-buten-1-al mit geschützter Aldehydgruppe mit einer acylierten Zuckereinheit so vorteilhaft durchführen läßt, daß sich durch Kombination dieses Glykosylierungsschrittes mit der Wittig-Reaktion mit einem β-Jonylidentriphenylphosphonium-Derivat ein sehr vorteilhaftes Gesamtverfahren zur Herstellung von Retinylglykosiden ergibt.

Gegenstand der Erfindung ist daher ein Verfahren zur Herstellung von Retinylglykosiden der allgemeinen Formel I

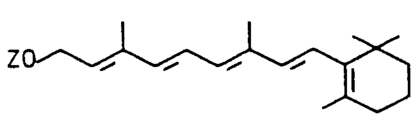

(I),

in der Z für einen geradkettigen oder verzweigten glykosidischen Rest enthaltend 1 bis 20, vorzugsweise 1 bis 3, insbesondere 1 bis 2 glykosidische Einheiten pro Rest steht, durch Glykosidierung eines vollständig acylierten Kohlenhydrats oder vollständig acylierten glykosidischen Polymers der allgemeinen Formel II

Z (acyliert)-Y (II),

in dem Y für eine für Glykosidierungen übliche Abgangsgruppe, wie F, Cl, Br oder der Rest

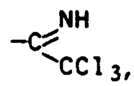

vorzugsweise für Cl oder Br, in 1-Stellung des Kohlenhydrats bzw. in 1-Stellung der endständigen Glykosideinheit steht, und in dem die H-Atome der freien OH-Gruppen des acylierten Glykosids bzw. acylierten glykosidischen Polymers durch einen Acylrest -CO-$R_1$ substituiert sind, worin $R_1$ für einen Alkylrest mit 1 bis 10 C-Atomen, vorzugsweise 1 oder 2 C-Atomen, oder einen Aralkylrest, vorzugsweise den Benzylrest steht, das dadurch gekennzeichnet ist, daß man

A. das acylierte Kohlenhydrat oder glykosidische Polymer mit einem an der Aldehydgruppe geschützten 4-Hydroxy-2-methyl-2-buten-1-al der allgemeinen Formel III

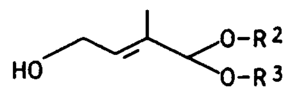 (III),

in der $R^2$ und $R^3$ für einen Alkylrest mit 1 bis 10, vorzugsweise 1 bis 4 C-Atomen, insbesondere eine Methylgruppe oder Ethylgruppe, oder einen Aralkylrest mit 7 bis 10 C-Atomen oder eine Phenylgruppe stehen oder $R^2$ und $R^3$ zusammen für einen Ethylen- oder Propylenrest, die mit Alkylgruppen mit 1 bis 4 C-Atomen, vorzugsweise einer oder mehreren Methylgruppen substituiert sein können, insbesondere den 2,2-Dimethylpropylenrest stehen, unter den für Glykosidierungen üblichen Bedingungen umsetzt,

B. aus der dabei erhaltenen Verbindung der allgemeinen Formel IV

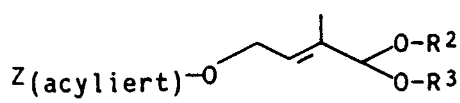 (IV)

die Aldehydschutzgruppe sauer abspaltet,

C. den dabei erhaltenen Aldehyd der allgemeinen Formel V

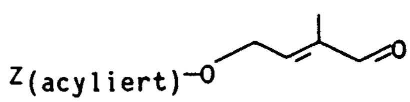 (V)

unter den Bedingungen einer Wittig-Reaktion mit einem $\beta$-Jonylidenethyl-triphenylphosphoniumsalz der allgemeinen Formel VI

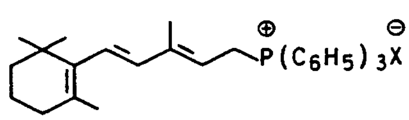 (VI),

in der X für ein einwertiges Anion, insbesondere für Cl, Br oder $HSO_4$ steht, umsetzt und

D. aus dem erhaltenen acylierten Retinylglykosid der allgemeinen Formel VII

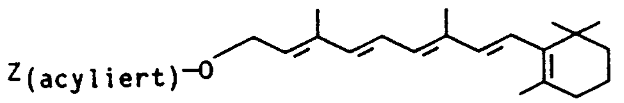

(VII)

in an sich bekannter Weise die Acylgruppen des Glykosidrestes abspaltet.

Von besonderer Bedeutung ist das erfindungsgemäße Verfahren für die Herstellung vom Retinylglykosiden der allgemeinen Formel Ia

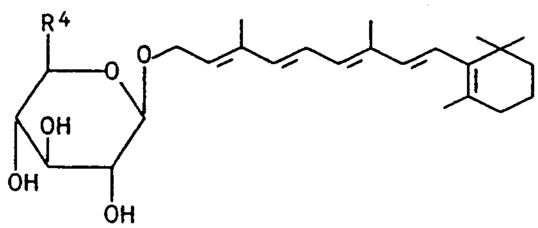

(Ia),

in der $R^4$ für $-CH_2-OH$, $-COOH$ oder $-COOCH_3$ steht,
bei dem

A. ein acyliertes Glykosid der allgemeinen Formel II

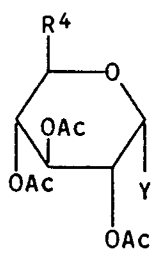

(II),

in der $R^4$ für $-CH_2-OCOCH_3$ oder $-COOCH_3$ steht,
Y für F, Cl, Br oder den Rest

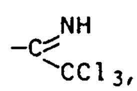

,

insbesondere für Br, steht und
Ac für einen Acylrest mit 1 bis 11 C-Atomen, vorzugsweise eine Acetylgruppe, oder einen Arylcarbonylrest, vorzugsweise den Benzoylrest steht, mit dem an der Aldehydgruppe geschützten 4-Hydroxy-2-methyl-2-buten-1-al der allgemeinen Formel III umsetzt,

B. aus der erhaltenen Verbindung der allgemeinen Formel IVa

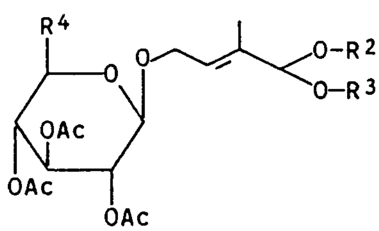

(IVa),

in der $R^2$, $R^3$ und Ac die oben angegebene Bedeutung haben und $R^4$ für $-CH_2-OCOCH_3$ oder $-COOCH_3$

steht, die Aldehydschutzgruppe abspaltet,
C. den dabei erhaltenen Aldehyd der allgemeinen Formel Va

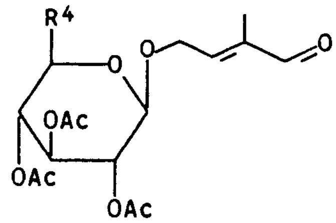

(Va),

mit einem β-Jonyliden-ethyl-triphenylphosphoniumsalz der allgemeinen Formel VI umsetzt und
D. aus dem dabei erhaltenen Retinylglykosid der allgemeinen Formel VIIa

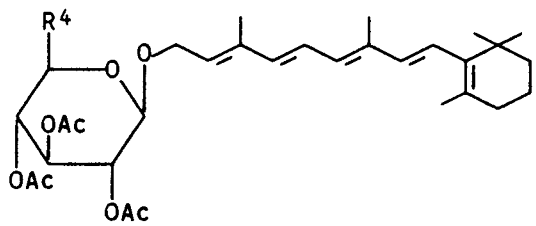

(VIIa),

die Schutzgruppen im glykosidischen Rest in an sich bekannter Weise abspaltet und gewünschtenfalls die razemische Verbindung VIIa in sein cis- und sein trans-Isomeres auftrennt.

Gegenstand der Erfindung sind außerdem Zwischenprodukte, die das außerordentlich vorteilhafte erfindungsgemäße Verfahren erst ermöglichen. Genannt seien
1-O-(2'-Methyl-2'-buten-4'-yl-1'-al-neopentylglykolacetal)-2,3,4,6-tetra-O-acetyl-β-D-glucopyranosid und Methyl-[1-O-(2'-methyl-2'-buten-4'-yl-1'-al-neopentylglykolacetal)-2,3,4-tri-O-acetyl-β-D-glucopyranosid]-uronat (beides Verbindungen der allgemeinen Formel IVa, in der $R^2$ und $R^3$ zusammen für den Rest $-CH_2-C(CH_3)_2-CH_2-$ stehen, Ac einen Acetylrest und $R^3$ den Rest $-CH_2-O-CO-CH_3$ bzw. $-COOCH_3$ bedeutet) sowie
1-O-(2'-Methyl-2'-buten-4'-yl-1'-al)-2,3,4,6-tetra-O-acetyl-β-D-glucopyranosid und Methyl-[1-O-(2'-methyl-2'-buten-4'-yl-1'-al)-2,3,4-tri-O-acetyl-β-D-gluco-pyranosid]-uronat (beides Verbindungen der allgemeinen Formel Va, in der Ac einen Acetylrest und $R^3$ den Rest $-CH_2-O-CO-CH_3$ bzw. $-COOCH_3$ bedeutet).

Gegenstand der Erfindung ist ferner die Verwendung der Zwischenprodukte der allgemeinen Formeln IV, IVa, V und Va zur Herstellung von Arzneimitteln. Bezüglich näherer Einzelheiten über Herstellung und Einsatz von aus Retinylglykosiden hergestellten Arzneimitteln verweisen wir beispielsweise auf F.M. Kaspersen, Xenobiotica 17 (1987), 1451-71 und G.A. Pitt, Biochem. Soc. Trans. 14 (1986) 923-984.

Mit dem Ausdruck glykosidische Einheiten werden sowohl Glykopyranosyl- als auch Glykofuranosyleinheiten, sowie deren Aminozuckerderivate oder Uronsäureeinheiten bezeichnet. In sogenannten glykosidischen Polymeren ist eine oder mehrere der $O-COR^1$-Gruppen im Kohlenhydratring ersetzt durch eine vollständig acylierte glykosidische Einheit mit der Maßgabe, daß die Anzahl der glykosidischen Einheiten 20 nicht übersteigt. Die Reihenfolge der glykosidischen Einheiten im Polymer ist ohne Einfluß auf das erfindungsgemäße Verfahren.

Als Kohlenhydrate bzw. glykosidische Polymere bezeichnen wir im Rahmen dieser Erfindung Monosaccharide, d.h. Pentosen und Hexosen in Pyranoseform oder Furanoseform, wie insbesondere Glucose, Mannose und Galactose; Disaccharide, wie Lactose, Maltose, Gentiobiose, Saccharose und Cellobiose; Polysaccharide, wie Maltotriose, Maltodextrin und Glucane, Aminohexosen, wie Glucosamin, Galaktosamin oder Mannosamin sowie Hexuronsäuren, wie Glucuronsäure, Galakturonsäure, Mannuronsäure oder 2-Keto-L-gulonsäure.

Vor der Glykosidierung müssen die funktionellen Gruppen der Glykoside oder glykosidischen Polymere geschützt werden. Als Schutzgruppen sind solche geeignet, die mit Basen leicht wieder abgespalten werden können. Von besonderer Bedeutung für den Schutz freier OH-Gruppen sind die Acetylgruppe, die Propionylgruppe und die Benzoylgruppe. Als geeignete Schutzgruppen für die COOH-Gruppen der Glucuronsäuren seien insbesondere die Methyl-, Ethyl- und Benzylgruppe genannt (vgl. H. Hulbuch, Kontakte 3/79, Seiten 14 bis 23).

Als geeignete Schutzgruppen für die Aminogruppen der Aminohexosen sind besonders geeignet organische Acylreste mit 2 bis 10 Kohlenstoffatomen, wie der Acetyl-, Propionyl- oder Butyrylrest, insbesondere der Acetylrest.

Als für Glykosidierungen übliche Abgangsgruppen seien genannt: F, Cl, Br und

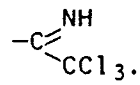

Von besonderer Bedeutung als Abgangsgruppe sind die Halogene, insbesondere Cl und Br. Die acylierten Glykoside enthaltend eine geeignete Abgangsgruppe in 1-Stellung des ersten (oder einzigen) glykosidischen Rings erhält man beispielsweise gemäß Methoden, die in "Methods in Carbohydrate Chemistry", Vol II (1963), Seiten 221 bis 222 und 228ff oder im J. Organ. Chem. 26 (1961), Seiten 908 bis 911 beschrieben sind.

Als an der Aldehydgruppe geschützte 4-Hydroxy-2-methyl-2-buten-1-ale der allgemeinen Formel III wird erfindungsgemäß insbesondere das 4-Hydroxy-2-methyl-2-buten-1-al-neopentylglykolacetal eingesetzt.

Die Glykosidierung mit den Alkoholen der Formel III ist unproblematisch und kann daher nach allen in der Kohlenhydratchemie üblichen Methoden durchgeführt werden. Einzelheiten über Glykosidierungsverfahren finden sich in der im folgenden genannten Literatur, welche hiermit als in die Anmeldung aufgenommen gilt:
Whistler, Wolfrom, "Methods in Carbohydrate Chemistry", Academic Press, Vol II (1963), Seiten 326 bis 366, Vol VIII (1980), Seiten 233 bis 261, sowie
John F. Kennedy, "Carbohydrate Chemistry", Clarendon Press, 1988, Seiten 500 bis 552.

Mit besonderem Vorteil gelingt die Glykosidierung in Gegenwart von Silbersalzen, $Hg(CN)_2$, $HgBr_2$; Lewissäuren, wie $BF_3$, $SnCl_4$ oder $TiCl_4$ in aprotischen Lösungsmitteln, wie Dimethylformamid (DMF), Hexan, Methylenchlorid, Toluol, Petrolether oder Dichlorethan. Die Reaktionstemperatur beträgt dabei im allgemeinen -40 bis 100 °C, vorzugsweise -20 °C bis Raumtemperatur. Der Fortgang der Reaktion kann dünnschichtchromatographisch beobachtet werden. Die Reinigung des Reaktionsproduktes kann säulenchromatographisch an Kieselgelen oder durch Kristallisation aus Alkanolen, insbesondere Methanol erfolgen.

Die Abspaltung der Aldehydschutzgruppe erfolgt in an sich bekannter Weise. Acetalgruppen beispielsweise werden gespalten durch Erwärmen in Gegenwart von sauren Katalysatoren. (Siehe beispielsweise T.W. Greene, Protective Groups in Organic Synthesis, John Wiley & Sons, 1981).

Zur Durchführung der Wittig-Reaktion werden etwa äquimolare Mengen des glykosidierten Aldehyds der Formeln V bzw. Va und des $\beta$-Jonylidenethyltriphenylphosphoniumsalzes in einem Gemisch aus einem für Wittig-Reaktionen üblichen unpolaren Lösungsmittel, wie Methylenchlorid, Benzol, Toluol, Chloroform, 1,2-Dichlorethan, Hexan, Cyclohexan, Petrolether und Wasser bei Temperaturen von etwa -20 bis +100 °C, vorzugsweise -5 bis Raumtemperatur unter intensiver Durchmischung mit einer starken Base, wie NaOH, KOH oder $NH_3$ versetzt. In einigen Fällen hat sich die Mitverwendung von Phasentransferkatalysatoren bei der Wittig-Reaktion als vorteilhaft erwiesen.

Als geeignete Phasentransferkatalysatoren seien insbesondere genannt:
Tetraalkylammoniumsalze der allgemeinen Formel VIII

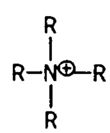 $X^{\ominus}$ (VIII),

in der die einzelnen R untereinander gleich oder verschieden sein können und für Alkylreste mit 1 bis 22 Kohlenstoffatomen oder für funktionelle Gruppen, wie Hydroxyl-, Acylamino- oder Alkoxygruppen enthaltende Alkylreste mit bis zu 25 Kohlenstoffatomen wie Methyl-, Ethyl-, (Iso)propyl-, Butyl-, Octyl-, Dodecyl-, $C_{16}H_{33}$-, Hydroxy(iso)propyl- oder

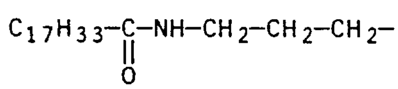

sowie für Phenylreste oder phenylsubstituierte Alkylreste (wie z.B. Benzyl-) mit bis zu 20 Kohlenstoffatomen stehen können,
und $X^{\ominus}$ für ein Anion einer Säure wie $J^-$, $Cl^-$, $Br^-$, $(HSO_4)^-$, $(CN)^-$, $(BF_4)^-$ steht; insbesondere das sehr preiswerte Trimethylbenzylammoniumchlorid, das in Form einer 50%igen wäßrigen Lösung eingesetzt werden kann, sowie Tricaprylmethylammoniumchlorid; und
Tetraalkylphosphoniumsalze der allgemeinen Formel IX

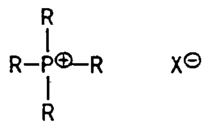

(IX),

worin die einzelnen R sowie $X^{\ominus}$ die für die Formel VIII angegebene Bedeutung haben, insbesondere das Tri-n-octyl-methylphosphoniumiodid.

Die Phasentransferkatalysatoren werden in Mengen von 0,01 bis 1, vorzugsweise 0,1 bis 0,5 Mol pro Mol Aldehyd eingesetzt.

Bezüglich näherer Einzelheiten über Wittig-Reaktionen verweisen wir auf H. Pommer, P.C. Thieme, Topics in Current Chemistry 109 (1985), Seiten 165 -188, bezüglich näherer Einzelheiten über Phasentransferkatalysatoren verweisen wir auf E.V. Dehmlow, Angewandte Chemie 89 (1977), Seiten 521ff sowie loc. cit. 86 (1974), Seiten 187ff.

Die Abspaltung der Schutzgruppen aus dem erhaltene acylierten Retinylglykosid der allgemeinen Formel VII bzw. VIIa kann in an sich bekannter Weise erfolgen, beispielsweise durch Behandeln mit Basen, wie Natriummethylat in Methanol, Ammoniak in Methanol oder mit einem stark basischen Ionenaustauscher, wie Amberlyst® A-26 (OH-Form) in Methanol.

Bei den erfindungsgemäß erhaltenen Retinylglykosiden handelt es sich um Razemate. Diese können anschließend beispielsweise mittels Säulenchromatographie an Kieselgelen in die cis- und trans-Isomeren gespalten werden. Diese Razemattrennung kann vor oder nach der Abspaltung der Schutzgruppen aus dem Retinylglykosid erfolgen. Besonders vorteilhaft ist es, die Razemattrennung vor Abspaltung der Schutzgruppen vorzunehmen.

Die Aufarbeitung des Reaktionsgemisches sowie die Reinigung der Reaktionsprodukte erfolgt auf übliche Weise, beispielsweise durch Filtration und Einengen des Filtrates und ggf. Chromatographie.

Mit Hilfe des erfindungsgemäßen Verfahrens können die Retinylglykoside der allgemeinen Formeln I und insbesondere Ia, in bedeutend besseren Ausbeuten erhalten werden, als gemäß dem Stand der Technik. Das erfindungsgemäße Verfahren gelingt auf einem Syntheseweg über neue interessante Zwischenprodukte.

Beispiel 1

A. Herstellung von 1-O-(2'-Methyl-2'-buten-1'-al-neopentylglykolacetal-4'-yl)-2,3,4,6-tetra-O-acetyl-β-D-glucopyranosid

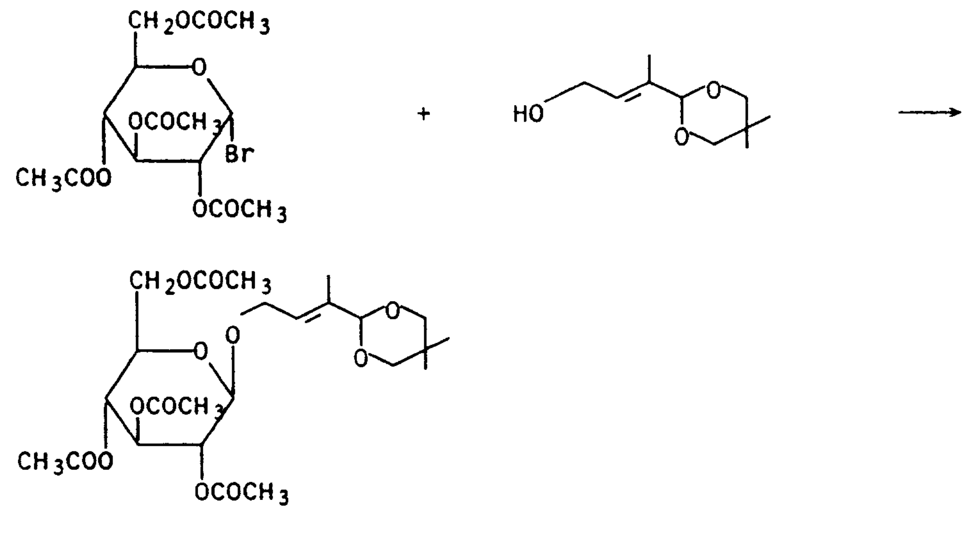

10,0 g (53,7 mmol) 4-Hydroxy-2-methyl-2-buten-1-al-neopentylglykolacetal, 5 g Molekularsieb (4Å pulverisiert) und 10 g (36,3 mmol) Silbercarbonat wurden in 10 ml absolutem (abs.) Methylenchlorid aufgenommen und das Reaktionsgemisch unter Argon-atmosphäre 15 Minuten (min) bei Raumtemperatur (RT) gerührt. Anschließend wurde eine Lösung von 14,7 g (35,8 mmol) Acetobromglucose in 10 ml $CH_2Cl_2$ innerhalb von 30 min in das Reaktionsgemisch getropft. Dann wurde das Reaktionsgemisch noch bis zum vollständigen Umsatz gerührt, wozu ca. 12 h notwendig waren. Der Fortgang der Umsetzung wurde durch Dünnschichtchromatographie (DC) an Kieselgelplatten mit $CHCl_3$/Essigsäureethylester (EE) im Volumenverhältnis (VV) von 2/1 als Laufmittel (LM) kontrolliert. Nach Abschluß der Reaktion wurde mit $CH_2Cl_2$ verdünnt, über ein Membranfilter filtriert und danach unter vermindertem Druck bis zu sirupartiger Konsistenz eingeengt. Das Reaktionsprodukt wurde säulenchromatographisch (Adsorbens: Kieselgel KG 60 der Firma Merck; LM: Toluol/Methanol im VV 10:1) gereinigt. Auch eine Kristallisation des Wertproduktes aus Ethanol (ca. 3 ml/g) ist möglich. Ausbeute: 16,0 g:86,5% der Theorie (d.Th.)

$[\alpha]_D^{20}$ : -20,1° (C = 1; $CHCl_3$)

Fp: 98,5 bis 104,9°C.

B. Herstellung von 1-O-(2'-Methyl-2'-buten-4'-yl-1'-al)-2,3,4,6-tetra-O-acetyl-$\beta$-D.glucopyranosid

9,0 g (17,8 mmol) des gemäß A. hergestellten Glucopyranosids wurden in 25 ml einer 60 Gew.-%igen wäßrigen Essigsäure aufgenommen und auf 40°C erwärmt. Nach 15 min trat vollständige Lösung ein. Gemäß DC (Adsorbens: Kieselgel KG 60, Merck; LM: $CHCl_3$/EE im VV 1:1) war die Umsetzung nach weiteren 30 min beendet. Anschließend wurde unter vermindertem Druck bei einer Badtemperatur von maximal 40°C eingeengt und die noch verbliebene Essigsäure durch mehrmaliges Codestillieren mit Toluol entfernt. Der erhaltene sirupöse Rückstand wurde in 20 ml Ethanol aufgenommen und das Produkt zur Kristallisation gebracht. Ausbeute: 6,9 g entsprechend 92% d.Th.

$[\alpha]_D^{20}$ : -18,5° (C = 1 ; $CHCl_3$)

Fp: 102 bis 105°C.

C. Herstellung von 1-O-Retinyl-2,3,4,6-tetra-O-acetyl-$\beta$-D-glucopyranosid

5 g (11,6 mmol) des gemäß B. erhaltenen 1-O-(2'-Methyl-2'-buten-4'-yl-1'-al)-2,3,4,6-tetra-O-acetyl-$\beta$-D-glucopyranosids und 6,5 g (11,6 mmol) $\beta$-Jonyliden-ethyl-triphenylphosphoniumchlorid wurden unter $N_2$-atmosphäre in 100 ml Methylenchlorid gelöst und mit 75 ml Wasser überschichtet. Anschließend wurde das Reaktionsgemisch auf 0°C abgekühlt, mit 25 ml einer 1N wäßrigen NaOH-Lösung versetzt und dann intensiv mit einem Turborührer gerührt. Nach ca. 30 min war die Reaktion beendet (DC: Toluol/EE im VV 3/1). Die organische Phase wurde abgetrennt, mit Wasser gewaschen, über $MgSO_4$ getrocknet und unter vermindertem Druck eingeengt. Durch eine Kieselgelfraktionierung (Kieselgel KG 60, Merck; Laufmittel Toluol/EE 4/1) erhält man die gewünschte Verbindung in Form eines cis-trans-Gemisches in einer Ausbeute von

5,57 g entsprechend 76% d. Th.

Anschließend wurde das Gemisch säulenchromatographisch (Adsorbens: Kieselgel KG 60; LM: Toluol/EE im VV 7/1) aufgetrennt. Man erhielt

1. das cis-Isomere mit $[\alpha]_D^{20}$ : -26,3° (C = 1; $CHCl_3$)
2. das trans-Isomere mit $[\alpha]_D^{20}$ : -31,6° (C = 1; $CHCl_3$)

D. Herstellung von 1-O-Retinyl-$\beta$-D-glucopyranosid

a. cis-Isomeres

250 mg (0,4 mmol) des gemäß C. erhaltenen cis-Tetraacetyl-Isomeren wurden in 4 ml Methanol gelöst und bei RT mit 0,2 ml einer 0,1 N $NaOCH_3$-Lösung versetzt. Nach ca. 1,5 h (Kontrolle mittels DC; LM $CHCl_3$/Methanol im VV 10:1) wurde mit Lewatit CNP L F ($H^{\oplus}$) neutralisiert und unter vermindertem Druck eingeengt.

Ausbeute: 179 mg entsprechend 98% d. Th.

$[\alpha]_D^{20}$ : -21,4° (C = 1,5; Methanol).

b. trans-Verbindung

Analog Beispiel 1D.a. wurden 192 mg (0,3 mmol) des gemäß C. erhaltenen trans-Tetraacetyl-Isomeren umgesetzt. Man erhielt das gewünschte trans-1-O-Retinyl-$\beta$-D-glucopyranosid in einer Ausbeute von 136 mg, entsprechend 98% d. Th.

$[\alpha]_D^{20}$ : -43,0° (C = 2; Methanol).

Beispiel 2

A. Herstellung von Methyl-[1-O-(2'-methyl-2'-buten-1'-al-neopentylglykolacetyl-4'-yl)-2,3,4-tri-O-acetyl-$\beta$-D-glucopyranosid]-uronat

2,8 g (15,0 mmol) 4-Hydroxy-2-methyl-2-buten-1-al-neopentylglykolacetal, 5 g Molekularsieb (4Å, pulv.) und 3 g (10,9 mmol) Silbercarbonat wurden in 10 ml abs. $CH_2Cl_2$ aufgenommen und das Reaktionsgemisch unter Stickstoffatmosphäre bei RT 15 min gerührt. Anschließend wurde innerhalb von 30 min eine Lösung von 3,0 g (7,55 mmol) D-Glucuronsäuremethylester-1-bromid in 6 ml $CH_2Cl_2$ in das Reaktionsgemisch getropft. Nach ca. 12 h war die Umsetzung vollständig. Das Reaktionsgemisch wurde mit 50 ml $CH_2Cl_2$ verdünnt, über ein Membranfilter filtriert und dann unter vermindertem Druck eingeengt. Das Wertprodukt wurde säulenchromatographisch (Adsorbens: Kieselgel KG 60; Laufmittel $CHCl_3$/EE im VV von 9:1) gereinigt. Auch eine Kristallisation aus Ethanol ist zur Reinigung anwendbar.

Ausbeute: 3,6 g: 94% d. Th.

$[\alpha]_D^{20}$ : -32,8° (C = 1; $CHCl_3$)

Fp: 107°C

B. Herstellung von Methyl-[1-O-(2'-methyl-2'-buten-4'-yl-1-al)-2,3,4-tri-O-acetyl-$\beta$-D-glucopyranosid]-uronat

10,0 g (19,9 mmol) eines gemäß Beispiel 2A hergestellten Uronats wurden in 30 ml einer 60%igen wäßrigen Essigsäure aufgenommen und auf 40°C erwärmt. Nach ca. 10 min erhielt man eine klare Lösung und nach weiteren 30 min war die Umsetzung beendet. (DC; $CHCl_3$/EE im VV 2:1). Es wurde unter vermindertem Druck eingeengt und mehrmals, zur vollständigen Entfernung der Essigsäure, mit Toluol co-destilliert. Das verbleibende hellgelbe Öl wurde in Ethanol aufgenommen und der erhaltene Aldehyd zur Kristallisation gebracht.

Ausbeute: 7,2 g entsprechend 87% d. Th.

$[\alpha]_D^{20}$ : -31,8° (C = 1 ; $CHCl_3$)

Fp: 125-127,5°C

C. Herstellung von Methyl-(1-O-Retinyl-2,3,4-tri-O-acetyl-$\beta$-D-glucopyranosid)-uronat

5,0 g (12 mmol) des gemäß Beispiel 2B erhaltenen Aldehyds, 6,75 g (12 mmol) $\beta$-Jonyliden-ethyltriphenylphosphoniumchlorid wurden unter Stickstoffatmosphäre in 100 ml $CH_2Cl_2$ gelöst und mit 75 ml Wasser überschichtet. Danach wurde auf 0°C abgekühlt und nach Zugabe von 25 ml einer 1 N NaOH-Lösung intensiv gerührt (Turborührer). Nach 15 min war die Reaktion beendet (DC; T/EE im VV 3:1). Die organische Phase wurde abgetrennt, mit Wasser gewaschen und über $MgSO_4$ getrocknet.

Ausbeute: 5,68 g entsprechend 77% d. Th. an dem cis/trans Gemisch. Chromatographische Auftrennung des Gemisches an Kieselgel KG 60 mit Toluol/Essigester im VV 10/1 als Laufmittel ergab das cis- und trans-Isomere mit folgenden Daten:

1. cis $[\alpha]_D^{20}$ : -41,2° (C = 1;$CHCl_3$)
2. trans $[\alpha]_D^{20}$ : -46,9° (C = 1;$CHCl_3$)

## Patentansprüche

1. Verfahren zur Herstellung von Retinylglykosiden der allgemeinen Formel I

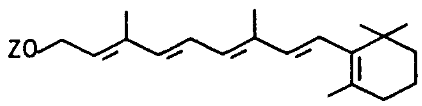

(I),

in der Z für einen geradkettigen oder verzweigten glykosidischen Rest enthaltend 1 bis 20 glykosidische Einheiten pro Rest steht, durch Glykosidierung eines vollständig acylierten Kohlenhydrats oder eines vollständig acylierten glykosidischen Polymers der allgemeinen Formel II

Z (acyliert)-Y (II),

in dem Y für eine für Glykosidierungen übliche Abgangsgruppe in 1-Stellung des Kohlenhydrats bzw. in 1-Stellung der endständigen Glykosideinheit steht, und in dem die H-Atome der freien OH-Gruppen des acylierten Glykosids bzw. acylierten glykosidischen Polymers durch einen Acylrest $-CO-R_1$ substituiert sind, worin $R_1$ für einen Alkylrest mit 1 bis 10-C-Atomen oder einen Aralkylrest steht, dadurch gekennzeichnet, daß man

A. das acylierte Kohlenhydrat oder glykosidische Polymer mit einem an der Aldehydgruppe geschützten 4-Hydroxy-2-methyl-2-buten-1-al der allgemeinen Formel III

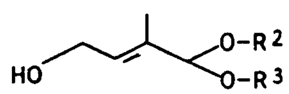

(III),

in der $R^2$ und $R^3$ für einen Alkylrest oder Aralkylrest mit bis zu 10 C-Atomen stehen oder $R^2$ und $R^3$ zusammen für einen Ethylenrest oder Propylenrest, die mit Alkylgruppen mit 1 bis 4 C-Atomen substituiert sein können, stehen, unter den für Glykosidierungen üblichen Bedingungen umsetzt,

B. aus der dabei erhaltenen Verbindung der allgemeinen Formel IV

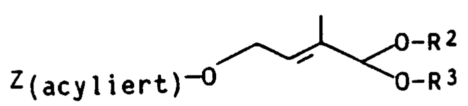

(IV)

die Aldehydschutzgruppe sauer abspaltet,

C. den dabei erhaltenen Aldehyd der allgemeinen Formel V

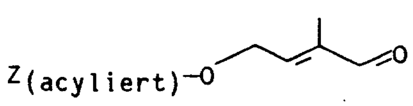

(V)

unter den Bedingungen einer Wittig-Reaktion mit einem $\beta$-Jonyliden-ethyl-triphenylphosphoniumsalz der allgemeinen Formel VI

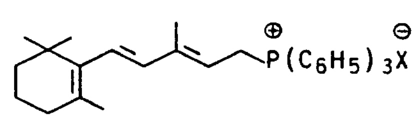

(VI),

in der X für ein einwertiges Anion steht, umsetzt und

D. aus dem erhaltenen acylierten Retinylglykosid der allgemeinen Formel VII

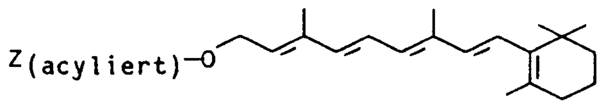

(VII)

in an sich bekannter Weise die Acylgruppen des Glykosidrestes abspaltet.

**2.** Verfahren zur Herstellung von Retinylglykosiden der allgemeinen Formel I gemäß Anspruch 1, dadurch gekennzeichnet, daß man als vollständig acyliertes Kohlenhydrat oder als vollständig acyliertes glykosidisches Polymer der allgemeinen Formel II

Z (acyliert)-Y (II),

ein solches verwendet, in dem Y für Cl oder Br als für Glykosidierungen übliche Abgangsgruppe steht.

**3.** Verfahren gemäß Anspruch 1 zur Herstellung von Retinylglykosiden der allgemeinen Formel Ia

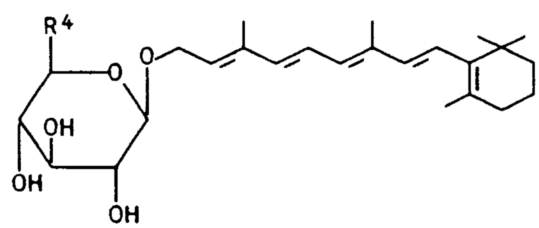

(Ia),

in der $R^4$ für $-CH_2-OH$, $-COOH$ oder $-COOCH_3$ steht, dadurch gekennzeichnet, daß man

A. ein acyliertes Glykosid der allgemeinen Formel IIa

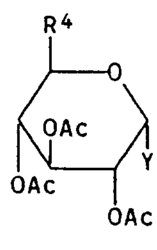

(IIa),

in der $R^4$ für $-CH_2-OCOCH_3$ oder $-COOCH_3$ steht, Y die oben angegebene Bedeutung hat und Ac für einen Acylrest mit 1 bis 11 C-Atomen, oder einen Arylcarbonylrest steht, mit dem Acetal des 4-Hydroxy-2-methyl-2-buten-1-als der allgemeinen Formel III umsetzt,

B. aus der erhaltenen Verbindung der allgemeinen Formel IVa

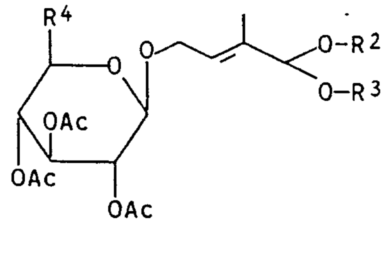

(IVa),

in der $R^2$ bis $R^3$ und Ac die oben angegebene Bedeutung haben und $R^4$ für $-CH_2-OCOCH_3$ oder

-$COOCH_3$ steht, die Aldehydschutzgruppe abspaltet,
C. den dabei erhaltenen Aldehyd der allgemeinen Formel Va

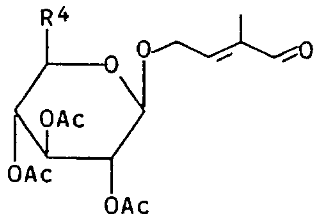

(Va),

mit dem $\beta$-Jonyliden-ethyl-triphenylphosphoniumsalz der allgemeinen Formel VI umsetzt und
D. aus dem dabei erhaltenen Retinylglykosid der allgemeinen Formel VIIa

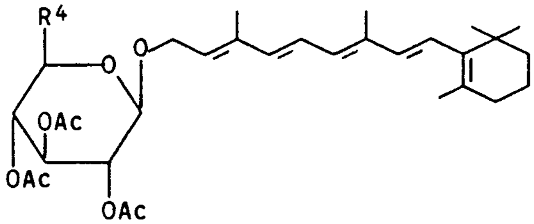

(VIIa),

die Schutzgruppen im glykosidischen Rest in an sich bekannter Weise abspaltet.

**4.** 1-O-(2'-Methyl-2'-buten-4'-yl-1'-al-neopentylglykolacetal)-2,3,4,6-tetra-O-acetyl-$\beta$-D-glucopyranosid

**5.** Methyl-[1-O-(2'-methyl-2'-buten-4'-yl-1'-al-neopentylglykolacetal)-2,3,4-tri-O-acetyl-$\beta$-D-glucopyranosid]-uronat

**6.** 1-O-(2'-Methyl-2'-buten-4'-yl-1'-al)-2,3,4,6-tetra-O-acetyl-$\beta$-D-glucopyranosid

**7.** Methyl-[1-O-(2'-methyl-2'-buten-4'-yl-1'-al)-2,3,4-tri-O-acetyl-$\beta$-D-gluco-pyranosid]-uronat

**8.** Verwendung der Verbindungen der allgemeinen Formel IV

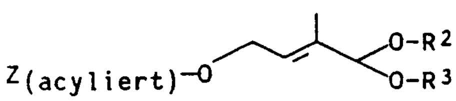

(IV)

in der Z, $R^2$ und $R^3$ die in Anspruch 1 angegebene Bedeutung haben, bzw. von Verbindungen der allgemeinen Formel IVa

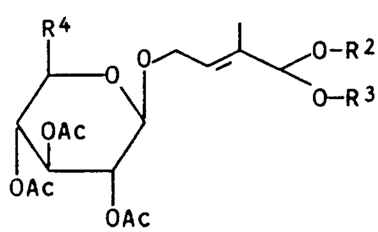

(IVa),

in der $R^2$, $R^3$, $R^4$ und Ac die in Anspruch 2 angegebene Bedeutung haben, zur Herstellung von Arzneimitteln

**9.** Verwendung von Aldehyden der allgemeinen Formel V

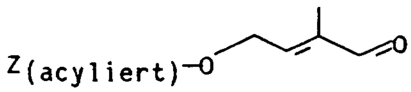

(V)

in der Z die in Anspruch 1 angegebene Bedeutung hat, bzw. von Aldehyden der allgemeinen Formel Va

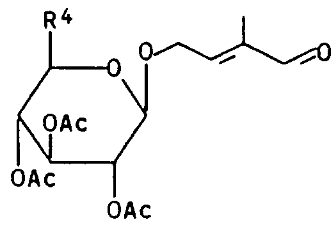

(Va),

in der $R^4$ und Ac die in Anspruch 2 angegebene Bedeutung haben, zur Herstellung von Arzneimitteln.

**Claims**

**1.** A process for preparing a retinyl glycoside of the formula I

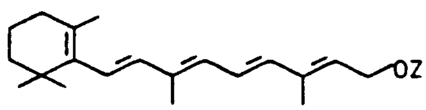

(I)

where Z is a straight-chain or branched glycosidic residue containing from 1 to 20 glycosidic units per residue, by glycosidation of a completely acylated carbohydrate or completely acylated glycosidic polymer of the formula II

Z (acylated)-Y (II)

where Y is a leaving group customary for glycosidations, in the 1-position of the carbohydrate or in the 1-position of the terminal glycoside unit, and where the H atoms of the free OH groups of the acylated glycoside or acylated glycosidic polymer have been replaced by -CO-$R_1$ where $R_1$ is alkyl of 1 to 10 carbon atoms or aralkyl, which comprises

A. reacting the acylated carbohydrate or glycosidic polymer with an aldehyde-protected 4-hydroxy-2-methyl-2-buten-1-al of the formula III

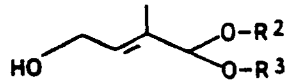

(III)

where $R^2$ and $R^3$ are each alkyl or aralkyl of up to 10 carbon atoms, or $R^2$ and $R^3$ are together ethylene or propylene, each of which can be substituted by alkyl groups of 1 to 4 carbon atoms, under the conditions customary for glycosidations,

B. eliminating the aldehyde protective group from the resulting compound of the formula IV

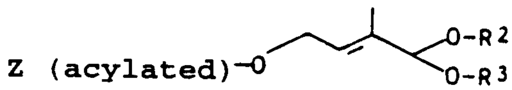

(IV)

with acid,
C. subjecting the resulting aldehyde of the formula V

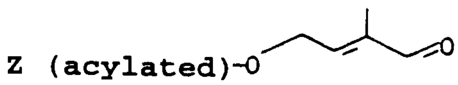

(V)

to a Wittig reaction with a $\beta$-ionylideneethyltriphenylphosphonium salt of the formula VI

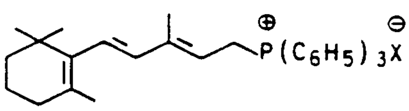

(VI)

where X is a singly charged anion, and
D. eliminating the acyl groups of the glycoside residue in the resulting acylated retinyl glycoside of the formula VII

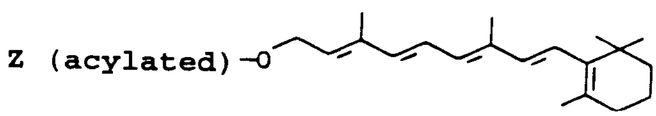

(VII)

in a conventional manner.

**2.** A process for preparing a retinyl glycoside of the formula I as claimed in claim 1, wherein the completely acylated carbohydrate or glycosidic polymer of the formula II

Z (acylated)-Y (II)

is one in which Y is Cl or Br.

**3.** A process as claimed in claim 1 for preparing a retinyl glycoside of the formula Ia

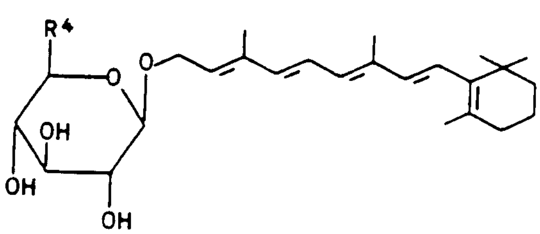

(Ia)

where $R^4$ is $-CH_2-OH$, $-COOH$ or $-COOCH_3$, which comprises

A. reacting an acylated glycoside of the formula IIa

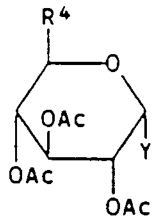

(IIa)

where $R^4$ is $-CH_2-OCOCH_3$ or $-COOCH_3$, Y has the above-mentioned meaning and Ac is acyl of 1 to 11 carbon atoms, or arylcarbonyl, with an acetal of 4-hydroxy-2-methyl-2-buten-1-al of the formula III,

B. eliminating the aldehyde protective group from the resulting compound of the formula IVa

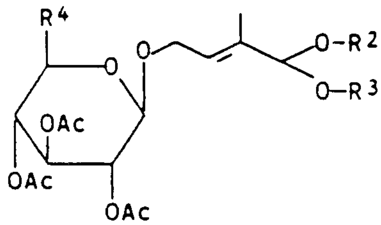

(IVa)

where $R^2$, $R^3$ and Ac have the abovementioned meanings, and $R^4$ is $-CH_2-OCOCH_3$ or $-COOCH_3$,

C. reacting the resulting aldehyde of the formula Va

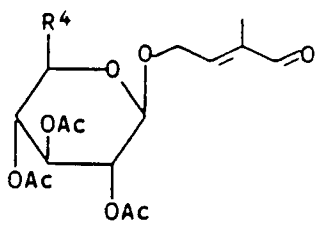

(Va)

with the $\beta$-ionylideneethyltriphenylphosphonium salt of the formula VI and

D. eliminating the protective groups in the glycosidic residue of the resulting retinyl glycoside of the formula VIIa

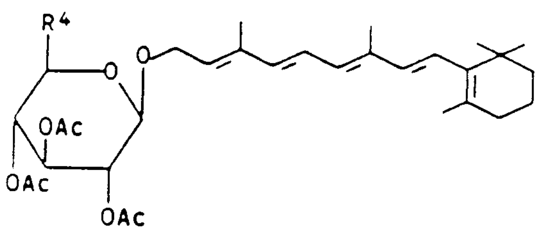

(VIIa)

in a conventional manner.

**4.** 1-O-(2'-Methyl-2'-buten-4'-yl-1'-al-neopentyl glycol acetal)-2,3,4,6-tetra-O-acetyl-$\beta$-D-glucopyranoside.

**5.** Methyl [1-O-(2'-methyl-2'-buten-4'-yl-1'-al neopentyl glycol acetal)-2,3,4-tri-O-acetyl-$\beta$-D-glucopyranosid]-uronate.

**6.** 1-O-(2'-Methyl-2'-buten-4'-yl-1'-al)-2,3,4,6-tetra-O-acetyl-$\beta$-D-glucopyranoside.

**7.** Methyl [1-O-(2'-methyl-2'-buten-4'-yl-1'-al)-2,3,4-tri-O-acetyl-$\beta$-D-gluco-pyranosid]-uronate.

**8.** The use of a compound of the formula IV

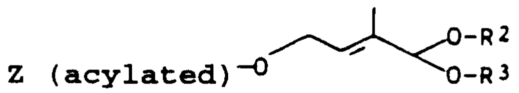

(IV)

where Z, $R^2$ and $R^3$ have the meanings stated in claim 1, or of a compound of the formula IVa

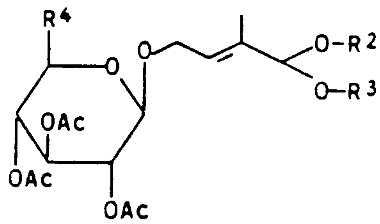

(IVa)

where $R^2$, $R^3$, $R^4$ and Ac have the meanings stated in claim 2, for the preparation of drugs.

**9.** The use of an aldehyde of the formula V

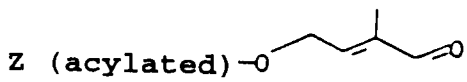

(V)

where Z has the meaning stated in claim 1, or of an aldehyde of the formula Va

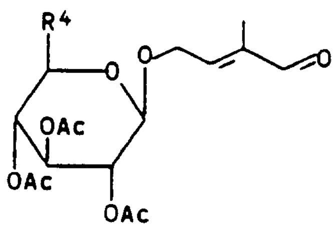

(Va)

where $R^4$ and Ac have the meanings stated in claim 2, for the preparation of drugs.

## Revendications

**1.** Procédé de préparation de rétinylglucosides de formule générale I

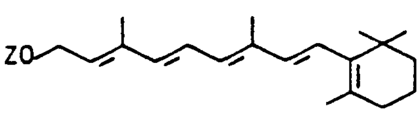

(I),

dans laquelle Z est mis pour un reste glucosidique en chaîne droite ou ramifiée contenant 1 à 20 motifs glucosidiques par reste, par glucosidation d'un glucide complètement acylé ou d'un polymère glucosidique complètement acylé de formule générale II

Z (acyle)-Y (II)

dans lequel Y est mis pour un groupement enlevable, usuel pour des glucosidations, en position 1 du glucide ou en position 1 du motif glucosidique terminal, et dans lequel les atomes d'hydrogène des groupements OH libres du glucoside acyle ou du polymère glucosidique acylé sont substitués par un reste acyle -CO-$R_1$, $R_1$ étant mis pour un reste alkyle à 1-10 atomes de carbone ou pour un reste aralkyle, caractérise en ce que

A. l'on fait reagir le glucide ou le polymère glucosidique acylé, dans les conditions usuelles pour des glucosidations, avec un 4-hydroxy-2-méthyl-2-butène-1-al protégé sur le groupement aldéhyde, de formule générale III

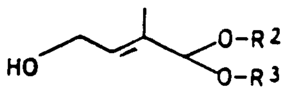

(III),

dans laquelle $R^2$ et $R^3$ sont mis chacun pour un reste alkyle ou un reste aralkyle contenant jusqu'a 10 atomes de carbone ou $R^2$ et $R^3$ sont mis ensemble pour un reste éthylène ou un reste propylène qui peut être substitué par des groupements alkyle à 1-4 atomes de carbone,

B. on élimine par acide le groupement protecteur d'aldéhyde du composé de formule générale IV ainsi obtenu

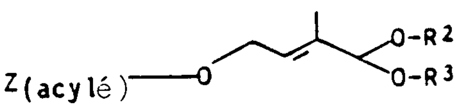

(IV)

C. on fait réagir l'aldéhyde de formule générale V ainsi obtenu

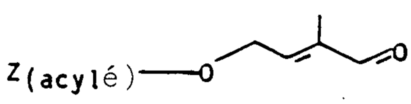

(V)

dans les conditions d'une reaction de Wittig, avec un sel de $\beta$-ionylidène-éthyl-triphénylphosphonium de formule générale VI

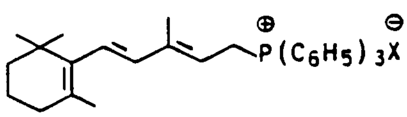

(VI),

dans laquelle X est mis pour un anion monovalent, et

D. on élimine les groupements acyle du reste glucosidique, de façon connue en soi, du retinylglucoside acylé de formule générale VII ainsi obtenu

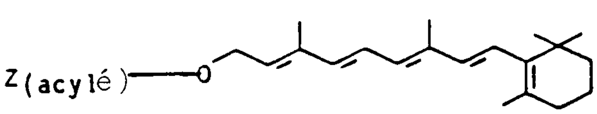

(VII)

**2.** Procédé de préparation de retinylglucosides de formule générale I selon la revendication 1, caractérisé en ce qu'on utilise, comme glucide complètement acylé ou comme polymère glucosidique complètement acylé de formule générale II

Z (acylé)-Y (II)

un composé dans lequel Y est mis pour Cl ou Br en tant que groupement enlevable, usuel pour des glucosidations.

**3.** Procédé selon la revendication 1 pour la préparation de retinylglucosides de formule générale Ia

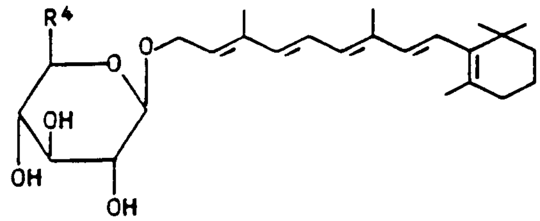

(Ia),

dans laquelle $R^4$ est mis pour $-CH_2-OH$, -COOH ou $-COOCH_3$, caractérisé en ce que

A. l'on fait réagir un glucoside acylé de formule générale IIa

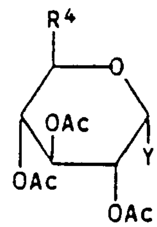

(IIa),

dans laquelle $R^4$ est mis pour $-CH_2-OCOCH_3$ ou $-COOCH_3$, Y a la signification indiquée précédemment et Ac est mis pour un reste acyle à 1-11 atomes de carbone ou pour un reste arylcarbonyle, avec l'acétal du 4-hydroxy-2-méthyl-2-butène-1-al de formule générale III,

B. on élimine le groupement de protection d'aldéhyde du composé obtenu de formule IVa

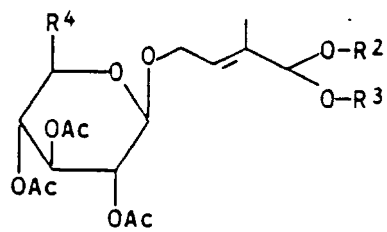

(IVa),

dans laquelle $R^2$, $R^3$ et Ac ont les significations indiquées précédemment et $R^4$ est mis pour $-CH_2-OCOCH_3$ ou $-COOCH_3$,

C. on fait réagir l'aldéhyde de formule générale Va ainsi obtenu

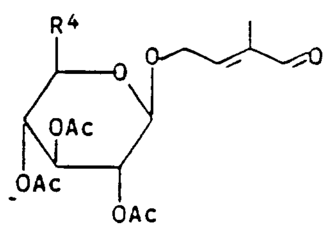

(Va),

avec le sel de β-ionylidène-éthyltriphénylphosphonium de formule générale VI et
D. on élimine les groupements de protection dans le reste glucosidique, de façon connue en soi, du rétinylglucoside de formule générale VIIa ainsi obtenu

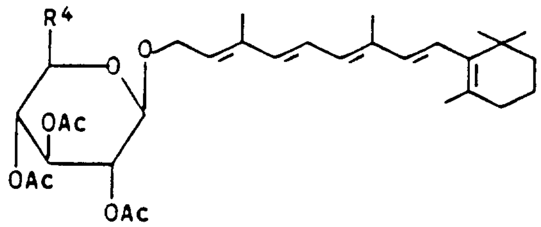

(VIIa),

4. 1-O-(2'-Methyl-2'-butène-4'-yl-1'-al-néopentylglycolacetal)-2,3,4,6-tétra-O-acétyl-β-D-glucopyranoside

5. Méthyl-[1-O-(2'-méthyl-2'-butène-4'-yl-1'-al-néopentylglycolacétal)-2,3,4-tri-O-acetyl-β-D-glucopyranoside]-uronate.

6. 1-O-(2'-Méthyl-2'-butène-4'-yl-1'-al)-2,3,4,6-tétra-O-acétyl-β-D-glucopyranoside.

7. Méthyl-[1-O-(2'-méthyl-2'-butène-4'-yl-1'-al)-2,3,4-tri-O-acétyl-β-D-glucopyranoside]-uronate.

8. Utilisation des composés de formule générale IV

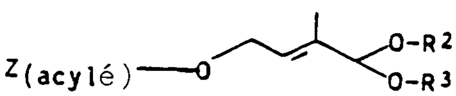

(IV)

dans laquelle Z, $R^2$ et $R^3$ ont les significations indiquées dans la revendication 1, ou de composés de formule générale IVa

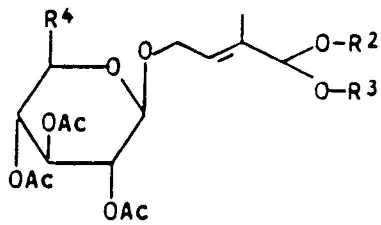

(IVa),

dans laquelle $R^2$, $R^3$, $R^4$ et Ac ont les significations indiquées dans la revendication 2, pour la préparation de médicaments.

9. Utilisation d'aldéhydes de formule générale V

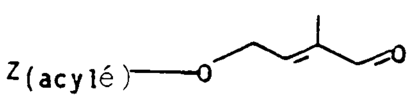

(V)

dans laquelle Z a la signification indiquée dans la revendication 1, ou d'aldéhydes de formule générale Va

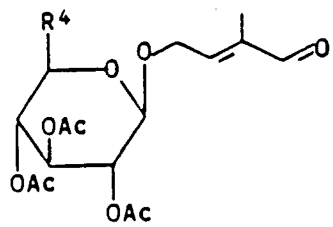

(Va),

dans laquelle $R^4$ et Ac ont les significations indiquées dans la revendication 2, pour la préparation de médicaments.